# EUROPÄISCHE PATENTSCHRIFT

(11) **EP 2 582 364 B1**
(45) Veröffentlichungstag und Bekanntmachung des Hinweises auf die Patenterteilung: **01.05.2019**
(21) Anmeldenummer: 11726705.4
(22) Anmeldetag: 15.06.2011
(51) Int. Cl.: A61K 9/70, A61K 31/13

(54) **TRANSDERMALE VERABREICHUNG VON MEMANTIN**
TRANSDERMAL ADMINISTRATION OF MEMANTINE
ADMINISTRATION TRANSDERMIQUE DE MÉMANTINE

(30) Priorität: 17.06.2010 DE 102010024105
(43) Veröffentlichungstag der Anmeldung: 24.04.2013
(73) Patentinhaber: LTS LOHMANN Therapie-Systeme AG, 56626 Andernach (DE); Grünenthal GmbH, 52078 Aachen (DE)
(72) Erfinder: HORSTMANN, Michael, 56564 Neuwied (DE); EIFLER, René, 56072 Koblenz (DE); KAUFMANN, Regine, 56567 Neuwied (DE); MOHR, Patrick, 53498 Bad Breisig (DE); GALIA, Eric, 52078 Aachen (DE); PRANGE, Wolfgang, 53501 Grafschaft (DE); BULLER, Stefan, 52499 Baesweiler (DE); PUSECKER, Klaus, 41352 Korschenbroich (DE); STAMPFUSS, Jan, 40221 Düsseldorf (DE); STOELBEN, Susanne, 50933 Köln (DE)
(74) Vertreter: Schweitzer, Klaus
(86) Internationale Anmeldenummer: PCT/EP2011/002953
(87) Internationale Veröffentlichungsnummer: WO 2011/157416

(56) Entgegenhaltungen:
- WO-A1-2005/072705
- WO-A1-2009/112167
- JP-A- 2009 013 171

## Beschreibung

Die Erfindung betrifft Transdermale Therapeutische Systeme (TTS), welche als Wirkstoff den NMDA Rezeptor Antagonisten Memantin oder eines seiner physiologisch verträglichen Salze enthalten. Die Transdermalen therapeutischen Systeme können zur Behandlung von Alzheimer hergestellt und eingesetzt werden.

Nach dem Stand der Technik werden Erkrankungen des Zentralnervensystems, insbesondere Demenzerkrankungen vom Typ Alzheimer in seiner leichten bis mittelschweren Form durch Acetylcholinesterase Inhibitoren (z.B. Donepezil) behandelt. Zur Behandlung von mittelschwerer bis schwerer Alzheimererkrankung werden NMDA Rezeptor Antagonisten eingesetzt.

Die Entstehung der Alzheimerschen Erkrankung beruht auf neurodegenerativen Prozessen mit Zellschwund im Cortex bzw. Hirnregionen. Cholinerge Nervenbahnen inklusive deren Projektionsgebiete sind dabei in besonderem Maße betroffen. Im Frühstadium der Erkrankung ist das glutamaterge System gestört. Infolge eines übermäßigen Calcium-Einflusses in die Neurone kann es langfristig zu einer Neurodegeneration kommen. Als wichtigste glutamaterge Synapse spielt der NMDA Komplex dabei eine zentrale Rolle. Seine Hemmung durch einen selektiven Antagonisten kann die neuronalen Gebiete vor exzitatorischen Schädigungen bewahren.

Zeichen einer Alzheimerschen Erkrankung sind stark erweiterte Ventrikel, mikroskopisch nachweisbare Plaques und neurofibrilläre Knötchen. Das klinische Bild ist meist durch Gedächtnisschwund sowie durch einen allmählichen Verfall der Persönlichkeit und des Intellekts geprägt. Insofern ist das Ziel einer Alzheimer Behandlung, persönliches Leid zu mildern und somit einer sozialen Ausgrenzung entgegenzuwirken. Eine kurative Therapie ist derzeit nicht verfügbar. Insofern ist es gewünscht, die Entwicklung der Demenz, die meist progressiv verläuft, entsprechend hinauszuzögern. Eine palliative Therapie hat dabei die folgenden Ziele: Verbesserung der kognitiven Leistungsfähigkeit, Minimierung auffälliger neurologischer Verhaltensmuster, Hinauszögern des Fortschreitens der Erkrankung und Verhinderung der Anfallssymptome.

Nach dem Stand der Technik ist bekannt, dass sich NMDA Rezeptor Antagonisten als Neuroprotektoren eignen, indem sie die schädliche Auswirkung von Glutamat an NMDA Rezeptoren verhindern. Zur Gruppe der NMDA Rezeptor Antagonisten gehören beispielsweise Substanzen wie MK 801, Dextromethorphan, Ketamin, Memantin, Amantadin, Dextrophan, Felbamat, Acamprosat, MRZ 2/579, Phencyclidin, Aptiganel, wobei diese sowohl als Einzelsubstanzen wie auch als Gemische bzw. als deren physiologisch verträglichen Salze, beispielsweise Hydrochloride, Citrate, Maleate usw. in Frage kommen.

Es ist bekannt, dass die Anwendung von NMDA Rezeptor Antagonisten insbesondere infolge psychomimetischer Nebeneffekte eingeschränkt ist, insofern ist deren breite Anwendung bislang nicht erfolgt. Besondere Bedeutung bei der Behandlung von Alzheimer Patienten kommt dabei dem NMDA Rezeptor Antagonisten Memantin (3,5-Dimethyl-9-Aminoadamantan) bzw. seinem Hydrochlorid zu. Memantin ist zur Behandlung von mittelschwerer bis schwerer Alzheimer Krankheit zugelassen. Das Medikament ist in Deutschland unter der Bezeichnung Axura® erhältlich. Das Arzneimittel steht in oraler Form von Tropfen zum Einnehmen oder als Filmtablette zur Verfügung. Die Verabreichung des Medikaments darf nur unter der Aufsicht eines Betreuers erfolgen, wobei die Wirkstoffdosis über einen Zeitraum von mehreren Wochen schrittweise gesteigert werden muss, um insbesondere die bei der Behandlung von mittelschweren bis schweren Alzheimererkrankungen beschriebenen Nebenwirkungen, wie u.a. Halluzinationen, Verwirrtheit, Schwindel, Kopfschmerzen und Müdigkeit, zu minimieren. Auch nach der Anfangsphase ist das Medikament streng jeweils einmal morgens und einmal nachmittags (Tropfen) bzw. bei Verwendung der Filmtabletten, täglich zur gleichen Zeit zu verabreichen.

Aufgrund dieses strengen Therapieschemas und der damit verbundenen Aufsichtspflicht ergibt sich die nachteilige Situation eines sehr hohen Aufwands bzw. hoher Therapiekosten bei der Betreuung dieser Patienten.

Aufgrund dieser genannten Nachteile ergibt sich ein Bedarf an neuen therapeutischen Systemen, welche diese Nachteile überwinden, insbesondere solcher Systeme, bei denen der Betreuungsaufwand reduziert werden kann.

Nach dem Stand der Technik ist bekannt, dass sich NMDA Rezeptor Antagonisten ggf. in Kombination mit Analgetika in topischen Darreichungsformen zur Behandlung von lokalen Schmerzen einsetzen lassen (WO 00/03716; WO 03/015 699; US 2002/0016319, US 6,194,000). Auch in US 2004/0102525, US 2003/0139698 und WO 04/009062 ist die Verabreichung von NMDA Rezeptor Antagonisten offenbart. WO 04/106275 offenbart spezielle Salze von NMDA Rezeptor Antagonisten.

Die WO 2005/072705 offenbart ein TTS, das Memantin zur Therapie von Alzheimer in kontrollierter Weise freigibt.

Die Darreichungsformen des Standes der Technik sind jedoch nicht oder nur bedingt dazu geeignet, nach topischer Applikation systemische Plasmakonzentrationen von NMDA Rezeptor Antagonisten aufzubauen, die einen ausreichenden pharmakologischen Effekt für eine ausreichende Dauer bewirken. Hinsichtlich einer transkutanen Permeabilität werden im Stand der Technik keine experimentellen Daten offenbart.

Bei der Vorbeugung oder Behandlung von Erkrankungen des Zentralnervensystems wären aber gerade Darreichungsformen besonders wünschenswert, welche pharmakologisch wirksame Plasmakonzentrationen über einen vergleichsweise - langen Zeitraum aufrechterhalten können (z.B. 12 h, 24 h oder länger), nicht zuletzt da in solchen Fällen der Betreuungsaufwand der Patienten durch Pflegepersonal reduziert werden könnte.

Der Erfindung liegt daher die Aufgabe zugrunde, eine Darreichungsform zur Behandlung oder Vorbeugung von Erkrankungen des Zentralnervensystems, insbesondere Demenzerkrankungen, vorzugsweise der Alzheimer-Erkrankung (Morbus Alzheimer) bereitzustellen, welche Vorteile gegenüber den Darreichungsformen des Standes der Technik aufweist.

Die Vorteile können dabei hinsichtlich der Anwendbarkeit, Dosierung und/oder Verträglichkeit zum Ausdruck kommen. Nach Möglichkeit sollte die Darreichungsform bei einmaliger Applikation pharmakologisch wirksame Plasmakonzentrationen des Wirkstoffs über einen längeren Zeitraum gewährleisten, beispielsweise für wenigstens 12 h, vorzugsweise für wenigstens 24 h oder sogar für mehrere Tage, so dass eine erneute Anwendung und damit auch eine erneute Kontrolle durch das Pflegepersonal weniger häufig erforderlich ist (z.B. nur einmal täglich).

Diese Aufgabe wird durch den Gegenstand der Patentansprüche gelöst.

Es wurde überraschend gefunden, dass ein Transdermales Therapeutisches System hergestellt werden kann, welches dazu geeignet ist, Memantin transdermal zu verabreichen, wobei die Anwendung beispielsweise für mindestens 12 h, oder z.B. über einen längeren Zeitraum, etwa für mindestens 24 h, erfolgen kann und während dieses Zeitraums pharmakologisch wirksame Plasmakonzentrationen von Memantin aufrechterhalten werden können.

Die Erfindung betrifft ein Transdermales Therapeutisches System (TTS) gemäß Anspruch 1 zur Verabreichung des Wirkstoffs Memantin in Form der freien Base oder eines physiologisch verträglichen Salzes oder Prodrugs, wobei das TTS
- eine wirkstoffundurchlässige Trägerschicht;
- mindestens eine wirkstoffhaltige Schicht, welche ein Speichermaterial enthält, in das der Wirkstoff eingebracht ist;
- gegebenenfalls eine Schutzfolie;
- eine tₘₐₓ von mindestens 24 h;
aufweist, und wobei bei aufeinanderfolgender oder zeitlich versetzter, bevorzugt wiederholter, Applikation jeweils eines oder mehrerer der Transdermalen Therapeutischen Systeme für Applikationsintervalle von jeweils unabhängig voneinander mindestens 12 h, vorzugsweise über einen Gesamtzeitraum von mindestens 300 h, am behandlungsbedürftigen Individuum in dessen Blutplasma jeweils ab Beginn der erstmaligen Applikation folgende Wirkstoffkonzentrationen erreicht werden:
nach 24 h mindestens 14 ng/ml, bevorzugt mindestens 16,2 ng/mL,
nach 48 h mindestens 27 ng/ml, bevorzugt mindestens 29,2 ng/mL,
nach 60 h mindestens 32 ng/ml, bevorzugt mindestens 34,5 ng/mL,
nach 72 h mindestens 37 ng/ml, bevorzugt mindestens 39,1 ng/mL,
nach 96 h mindestens 44 ng/ml, bevorzugt mindestens 46,6 ng/mL,
nach 144 h mindestens 54 ng/ml, bevorzugt mindestens 56,5 ng/mL,
nach 192 h mindestens 60 ng/ml, bevorzugt mindestens 62,3 ng/mL,
nach 240 h mindestens 63 ng/ml, bevorzugt mindestens 65,5 ng/mL, und
nach 300 h mindestens 65 ng/ml, bevorzugt mindestens 67,8 ng/mL.

Bevorzugt beträgt die maximale Freisetzungsrate des Wirkstoffs höchstens 100 µg/cm³. Das erfindungsgemäße TTS enthält den Wirkstoff Memantin (3,5-Dimethyl-adamantan-1-amin) in Form der freien Base oder eines physiologisch verträglichen Salzes oder Prodrugs. Zum Zwecke der Beschreibung umfasst der Begriff "Wirkstoff" Memantin und seine physiologisch verträglichen Solvate. Memantin kann dabei in Form der freien Base, in Form der physiologisch verträglichen Salze und/oder in Form der Solvate, insbesondere der Hydrate oder wahlweise in Form von beliebigen Gemischen der vorbezeichneten Verbindungen vorliegen.

In einer bevorzugten Ausführungsform enthält das erfindungsgemäße TTS Memantin als freie Base. Die prinzipielle Herstellung einer freien Base aus einem Hydrochlorid ist dem Fachmann bekannt. Diese kann beispielsweise unter Einsatz von Ionenaustauschern bewerkstelligt werden. Eine weitere Methode ist eine Extraktion aus alkalischem wässrigem Milieu unter Einsatz von organischen Lösungsmitteln, die üblicherweise mit Wasser nicht oder nur geringfügig mischbar sind. Durch Entfernen des organischen Lösemittels wird die freie Base (3,5-Dimethyl-adamantan-1-amin) erhalten.

In einer anderen bevorzugten Ausführungsform enthält das erfindungsgemäße TTS Memantin in Form eines physiologisch verträglichen Salzes. Bevorzugte physiologisch verträgliche Salze von Memantin sind beispielsweise sein Hydrochlorid, Hydrobromid, Sulfat, Hydrogensulfat, Phosphat, Hydrogenphosphat, Dihydrogenphosphat, Acetat, Propionat, Lactat, Citrat, Ascorbat, Formiat, Fumarat, Maleat, Sebacinat, Mesylat und Besylat. Memantin Hydrochlorid ist besonders bevorzugt.

Das erfindungsgemäße TTS kann als Wirkstoff neben Memantin bzw. seinen physiologisch verträglichen Salzen bzw. Prodrugs weitere pharmakologisch wirksame Substanzen enthalten. Bevorzugt sind jedoch neben Memantin bzw. seinen physiologisch verträglichen Salzen keine weiteren Wirkstoffe enthalten.

Bevorzugt ist das erfindungsgemäße TTS ein wirkstoffhaltiges Pflaster.

Üblicherweise umfasst ein solches TTS wenigstens
a) eine wirkstoffundurchlässige Trägerschicht (*backing layer*), bevorzugt ist eine Trägerschicht, die für Wasser in flüssiger Form und/oder Wasserdampf undurchlässig ist;
b) mindestens eine wirkstoffhaltige Schicht, welche die Trägerschicht zumindest teilweise bedeckt und vorzugsweise selbst haftklebend ausgerüstet ist. Gegebenenfalls kann eine zusätzliche haftklebende Schicht aufgebracht sein, um eine ausreichende Haftung auf der Haut zu gewährleisten;
c) ggf. eine Schutzfolie, welche die wirkstoffhaltige Schicht bedeckt und davon abziehbar ist (*release liner*). Bevorzugt ist eine Schutzfolie, die für Wasser in flüssiger Form und/oder Wasserdampf undurchlässig.

Die ggf. vorhandene Schutzfolie wird üblicherweise vor der Applikation des TTS entfernt, um die klebefähige Schicht freizulegen. Die Schutzfolie kann z.B. aus Polyethylen, Polyester, Polyethylenterephthalat, Polypropylen, Polysiloxan, Polyvinylchlorid oder Polyurethan und gegebenenfalls aus behandelten Papierfasern, wie z. B. Zellophan, bestehen und ggf. eine Silikon-, Fluorsilikon- oder Fluorcarbonbeschichtung aufweisen.

Das erfindungsgemäße TTS weist eine wirkstoffundurchlässige Trägerschicht und eine wirkstoffhaltige Schicht oder mehrere wirkstoffhaltige Schichten auf, wobei allerdings nicht alle Schichten neben der Trägerschicht wirkstoffhaltig sein müssen.

In einer bevorzugten Ausführungsform weist das erfindungsgemäße TTS insgesamt höchstens 3, 4, 5, 6, 7, 8 oder 9 verschiedene Schichten auf. In einer anderen bevorzugten Ausführungsform weist das erfindungsgemäße TTS insgesamt mindestens 3, 4, 5, 6, 7, 8 oder 9 verschiedene Schichten auf.

Die wirkstoffundurchlässige Trägerschicht (Rück- bzw. Deckschicht) des erfindungsgemäßen TTS ist vorzugsweise für die in der wirkstoffhaltigen Schicht und in der Klebeschicht enthaltenen Stoffe, insbesondere für den gegebenenfalls vorhandenen Wirkstoff undurchlässig und inert und kann aus Polymeren wie Polyester, z.B. Polyethylenphthalat, Polyolefinen, wie Polyethylenen, Polypropylenen oder Polybutylenen, Polycarbonaten, Polyethylenoxiden, Polyterephthalaten wie Polyethylenterephthalaten, Polyurethanen, Polystyrolen, Polyamiden, Polyimiden, Polyvinylacetaten, Polyvinylchloriden und/oder Polyvinylidenchloriden, Copolymeren wie Acrylonitril/Butadien/Styrol Copolymeren enthaltend Papierfasern, Textilfasern und/oder deren Mischungen aufgebaut sein, die bei Bedarf metallisiert oder pigmentiert sein können. Die Trägerschicht kann auch aus einer Kombination aus Metallfolie und Polymerschicht aufgebaut sein. Die aufgeführten Polymere können als Film, Gewebe, Filz oder Schaum eingesetzt werden.

Die wirkstoffundurchlässige Schicht weist vorzugsweise eine Schichtdicke im Bereich von 15 bis 2000 µm auf.

Das erfindungsgemäße TTS weist eine wirkstoffhaltige Schicht auf, welche ein Speichermaterial enthält, in das der Wirkstoff eingebracht ist. Diese wirkstoffhaltige Schicht kann gleichzeitig haftklebend sein, in dem der Wirkstoff gelöst und/oder dispergiert in einer Matrix zusammen mit dem Klebstoff vorliegt (*drug in adhesive*). Bei dem erfindungsgemäßen TTS können jedoch auch wirkstoffhaltige Schicht und Klebeschicht getrennt voneinander vorliegen.

Die wirkstoffhaltige Schicht weist-vorzugsweise eine Schichtdicke im Bereich von 10 bis 9000 µm auf.

Das erfindungsgemäße TTS kann beispielsweise als membrangesteuertes System (Reservoir-TTS) oder als diffusionsgesteuertes System (Matrix-TTS) ausgestaltet sein (vgl. K. H. Bauer, et al., Pharmazeutische Technologie; R. H. Müller, et al., Pharmazeutische Technologie: Moderne Arzneiformen).

Ein Reservoir-TTS umfasst üblicherweise einen auf der Haut selbstklebenden, flachen Beutel, welcher die Wirkstoffe gelöst enthält. Der Hautseite zugewandt ist der Beutel mit einer für den Wirkstoff durchlässigen Membran ausgerüstet, welche die Wirkstofffreigabe steuert. Klebefähige Schicht und wirkstoffhaltige Schicht (Reservoir) sind üblicherweise räumlich voneinander getrennte Untereinheiten.

Bei einem Matrix-TTS ist der Wirkstoff in einer Matrix eingebettet, wobei er in flüssigem, halbfestem oder festem Zustand dispergiert oder in gelöster Form vorliegen kann. Weist die Matrix gleichzeitig klebefähige Eigenschaften auf, so ist die klebefähige Schicht gleichzeitig auch die wirkstoffhaltige Schicht (*drug in adhesive*). Es ist aber auch möglich, dass die klebefähige Schicht und die wirkstoffhaltige Schicht - wie auch bei einem Reservoir-TTS - voneinander getrennt sind. In diesem Fall kann die klebefähige Schicht flächendeckend, partiell oder ringförmig auf der wirkstoffhaltigen Schicht bzw. der Membran des Reservoirs aufgebracht sein kann.

Durch die Zusammensetzung und Strukturierung der Matrix und/oder der Membran kann die Freisetzung des Wirkstoffs reguliert werden. Hinsichtlich weiterer Einzelheiten kann beispielsweise auf T.K. Gosh, Transdermal and Topical Drug Delivery Systems Es Into Practice, CRC Press, 1997; R.O. Potts et al., Mechanisms of Transdermal Drug Delivery (Drugs and the Pharmaceutical Sciences), Marcel Dekker, 1997; und R. Gurny et al, Dermal and Transdermal Drug Delivery. New Insights and Perspectives, Wissenschaftliche VG., Stuttgart, 1998 verwiesen werden.

Als Klebstoffe zur Herstellung der Klebeschicht des erfindungsgemäßen TTS können druckempfindliche Klebemittel (*pressure-sensitive adhesives,* PSA) eingesetzt werden. Die Klebeschicht kann beispielsweise als Klebebereich oder Klebefläche ausgebildet sein, welche die wirkstoffhaltige Schicht seitlich begrenzt. Zur Herstellung der Klebeschicht eignen sich beispielsweise Polymere wie Polyacrylate, Polyvinylether, Polyisobutylene (PIB), Styrol/Isopren- oder Butadien-/Styrol Copolymere oder Polyisopren Kautschuke. Weiterhin eignen sich Silikon-Klebstoffe, wie z. B. gegebenenfalls vernetzte Polydimethylsiloxane. Ferner sind Harze wie z. B. Ester von Glycinen, Glycerin oder Pentaerythrol, oder Kohlenwasserstoff-Harze wie Polyterpene geeignet. Klebstoffe auf Acrylatbasis werden durch Polymerisation von Acrylaten, Methacrylaten, Alkylacrylaten und/oder Alkylmethacrylaten, mit gegebenenfalls weiteren ungesättigten Monomeren wie Acrylamid, Dimethylacrylamid, Dimethylaminoethylacrylat, Hydroxyethylacrylat, Hydroxypropylacrylat, Methoxyethylacrylat, Methoxyethylmethacrylat, Acrylnitril und/oder Vinylacetat, hergestellt. Beispiele für geeignete Polymere sind kommerziell unter der Bezeichnung Durotak® erhältlich, beispielsweise die Produkte 2516, 2287, 900A oder 9301. Andere geeignete kommerziell verfügbare Polymere sind Polyisobutylene, Polyacrylate wie bespielsweise GMS 3083, Plastoid B und Eudragit® E 100.

Die Klebeschicht kann zusätzlich Hilfsstoffe wie Weichmacher, z.B. Phthalate wie Dibutylphthalate, Mineralöle, Ester von Zitronensäure, oder Ester von Glycerin, Hautdurchdringungsverstärker, z.B. Dimethylsulfoxid, Klebrigmacher (*tackifier*), Füllstoffe (wie Zinkoxid oder Silika), Vernetzer, Konservierungsmittel und/oder Lösungsmittel enthalten. Derartige Hilfsstoffe sind dem Fachmann bekannt. Diesbezüglich kann beispielsweise verwiesen werden auf R. Niedner et al., Dermatika: therapeutischer Einsatz, Pharmakologie und Pharmazie, Wiss. Verl.-Ges. 1992; H.P. Fiedler, Lexikon der Hilfsstoffe für Pharmazie, Kosmetik und angrenzende Gebiete, Editio Cantor Aulendorff, 2002.

Der Wirkstoff liegt in der wirkstoffhaltigen Schicht in gelöster oder in fester Form vor. Bevorzugt liegt der Wirkstoff in vollständig gelöster Form vor, d.h. es sind bevorzugt praktisch keine kristallinen Anteile des Wirkstoffs nachweisbar. In einer bevorzugten Ausführungsform beträgt die Konzentration des Wirkstoffs in der erfindungsgemäßen wirkstoffhaltigen Schicht mindestens 25%, bevorzugter mindestens 50%, noch bevorzugter mindestens 66%, am bevorzugtesten mindestens 75% und insbesondere aber mindestens 80% seiner Sättigungskonzentration. Die Bestimmung der Sättigungskonzentration ist dem Fachmann bekannt. Beispielsweise ist die Sättigungskonzentration noch nicht erreicht, wenn ein Kristall des reinen Wirkstoffs mit der wirkstoffhaltigen Schicht in Kontakt gebracht wird und innerhalb von 5 Minuten von dieser aufgenommen, d.h. gelöst wird. Liegt der Wirkstoff zumindest teilweise in fester Form vor, so handelt es sich vorzugsweise um eine Mikrodispersion.

Die wirkstoffhaltige Schicht des erfindungsgemäßen TTS enthält ein Speichermaterial, in das der Wirkstoff eingebracht ist.

In einer bevorzugten Ausführungsform ist das Speichermaterial zwischen der wirkstoffundurchlässigen Trägerschicht und einem wirkstoffdurchlässigen Membranmaterial angeordnet, so dass es sich bei dem erfindungsgemäßen TTS vorzugsweise um ein Reservoir-TTS handelt.

In einer anderen bevorzugten Ausführungsform dient das Speichermaterial als Matrix, so dass es sich bei dem erfindungsgemäßen TTS vorzugsweise um ein Matrix-TTS handelt.

Geeignete Speichermaterialien sind natürliche oder (halb-)synthetische matrixbildende Polymere. Als matrixbildende Polymere können z.B. genannt werden Polyethylene, chlorierte Polyethylene, Polypropylene, Polyurethane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylentherephthalate, Polytetrafluoroethylene, Ethylen/Propylen Copolymere, Ethylen/ Ethylacrylat Copolymere, Ethylen/Vinylacetat Copolymere, Ethylen/ Vinylalkohol Copolymere, Ethylen/ Vinyloxyethanol Copolymere, Vinylchlorid/ Vinylacetat Copolymere, Vinylpyrrolidon/Ethylen/Vinylacetat Copolymere, Kautschuke, Gummi-artige synthetische Homo-, Co- oder Blockpolymere, Silikone, Silikon-Derivate wie Polysiloxan/Polymethacrylat Copolymere, Cellulose-Derivate, wie Cellulose-Ether, insbesondere Methylcellulose, Ethylcellulose, Propylcellulose, Hydroxyethylcellulose, Hydroxypropylcellulose, Hydroxypropylmethylcellulose, Carboxymethylcellulose und/oder deren Mischungen. Polyacrylate oder Polysilikone sind besonders bevorzugt.

Das Speichermaterial kann die vorstehend genannten Polymere umfassen, welche zur Herstellung der Klebeschicht geeignet sind (*drug in adhesive*).

Der Gehalt an Speichermaterial beträgt bevorzugt 10 bis 99,9 Gew.-% bezogen auf das Gesamtgewicht der wirkstoffhaltigen Schicht, bevorzugter 50 bis 95 Gew.-%.

Hinsichtlich der Mengenverhältnisse Speichermaterial / Wirkstoffgehalt werden bevorzugt Verhältnisse von 99:1 bis 50:50 (Masse Speichermaterial : Masse Wirkstoff), vorzugsweise 95:5 bis 60:40 gewählt. In bevorzugten Ausführungsformen werden 99,5:0,5 bis 80:20 (Masse Matrix : Masse Wirkstoff), vorzugsweise 99:1 bis 85:15 gewählt. Der relativ hohe Anteil an Wirkstoff begründet sich am verhältnismäßig hohen Substanzbedarf zur Behandlung der mittelschweren bis schweren Alzheimerschen Erkrankung.

Bevorzugt umfasst das Speichermaterial
- eine flüssige oder gelförmige Zubereitung, in welcher der Wirkstoff teilweise oder vollständig gelöst ist; und/oder
- eine Matrix auf Basis eines polymeren Materials, in welcher der Wirkstoff gelöst oder als Mikrodispersion verteilt vorliegt; und/oder
- ein polymeres Material auf Polyacrylat- oder Polysilikonbasis; und/oder
- ein Lipogel, das vorzugsweise 10 bis 50 Gew.-% Wirkstoff enthält.

In einer bevorzugten Ausführungsform umfasst die wirkstoffhaltige Schicht ein Hydrogel oder ein Lipogel. Im Sinne der Beschreibung sind Hydrogele Gele, die Wasser und/oder andere hydrophile bzw. ganz oder teilweise mit Wasser mischbare Substanzen enthalten, die dem Gel die hydrophilen Eigenschaften verleihen. Im Sinne der Beschreibung sind Lipogele Gele, die hauptsächlich nicht mit Wasser mischbare Substanzen enthalten, die dem Gel die lipophilen Eigenschaften verleihen.

In einer bevorzugten Ausführungsform liegt der Wirkstoff in der wirkstoffhaltigen Schicht als physiologisch verträgliches Salz, insbesondere als Memantin Hydrochlorid, vor. Bevorzugt enthält die wirkstoffhaltige Schicht zusätzlich zumindest ein basisches Material, dessen Basizität ausreichend ist, um mit dem physiologisch verträglichen Salz des Wirkstoffs unter Freisetzung der freien Base des Wirkstoffs zu reagieren. Geeignete basische Materialien sind dem Fachmann bekannt und ihre Eignung kann auf Grundlage des pKₛ-Wertes der Memantin Base abgeschätzt werden. Vorzugsweise ist das basische Material ausgewählt aus der Gruppe bestehend aus Polymeren mit basischen Seitengruppe, organischen Basen und anorganischen Basen. In einer bevorzugten Ausführungsform ist das basische Material ausgesucht aus der Gruppe bestehend aus Polymeren auf Acrylatbasis, welche Aminoalkylseitengruppen aufweisen; anorganischen Oxiden oder Hydroxiden, insbesondere Alkali- oder Erdalkalioxiden; organischen Aminen oder deren Salzen; Alkali- oder Erdalkalialkoholaten oder -carboxylaten.

Bevorzugte Beispiele für geeignete basische Materialien sind Calciumoxid (CaO), Natriumethanolat und basische Methacrylat-Copolymere wie beispielsweise Eudragit® E100.

In einer bevorzugten Ausführungsform enthält die wirkstoffhaltige Schicht 20 bis 90 Gew.-% Speichermaterial, 10 bis 50 Gew.-% Wirkstoff, sowie ggf. 5 bis 40 Gew.-% basisches Material, bezogen auf das Gesamtgewicht der wirkstoffhaltigen Schicht.

Die wirkstoffhaltige Schicht des erfindungsgemäßen TTS kann neben dem Wirkstoff und dem ggf. vorhandenen Klebstoff auch Klebrigmacher, Weichmacher, Permeationsförderer, viskositätserhöhende Substanzen, Lösungsvermittler, Vernetzer, Konservierungsmittel, Emulgatoren, Verdickungsmittel und/oder andere übliche Hilfsmittel enthalten. Derartige Additive sind dem Fachmann bekannt.

Beispiele für Löslichkeitsverbesserer und/oder Permeationsförderer sind Acetylaceton, Acetyltributylcitrat, Acetyltriethylcitrat, Benzylalkohol, Butylstearat, Cetyllactat, Cetylpalmitat, Cetylstearat, Cetylstearylalkohol/Cetylalkohol, Chlorbutanol, Cineol, Decylmethylsulfoxid, Decyloleat, Dibutylphthalat, Diethylenglykolmoneethylether, Diethylphtahlat, Diethylsebacat, Diisopropyladipat, Dimethylphthalat, Dioctyladipat, Dipropylenglykol, Glycerylmonooleat, Glycerylmonostearat, Stearylalkohol, Erdnussöl, Ethyllactat, Ethyllinoleat, Ethyl-(9,12,15)-linolenat, Eugenol, Farnesol, Glycerin, Glycerylacetylester, Glycerylstearat, Glykoldistearat, Glyoxal, Hexadecanol, Hexylenglykol, Isobutylstearat, Isocetylstearat, Isodecyloleat, Isopropyllanolat, Isopropylmyristat/ Isopropylpalmitat, Isopropylstearat, Isostearylneopentanoat, Laurinsäurediethanolamid, Limonen, Linolensäure, Linolensäurediethanolamid, Minzcampher, Myristyllactat, Myristylmyristat, Myristylstearat, m-Tolylacetat, Octyldodecanol, Octylpalmitat, Octylstearat, Ölsäurediethanolamid, Oleylalkohol, Oleyloleat, Phenylethylalkohol, Propylenglykol, Propylencarbonat, Isostearinsäure, Octansäure, hydriertes Castoröl, 1,3-Butylenglykol, Distelöl, Squalan, Squalen, Triacetin, Glyceryltriacetat, Triethylcitrat, Undecylensäure, (+)-Fenchon, Ammoniumlaurylethersulfat, Cholesterin, Lecithin, Glycerinhydroxystearat, Mono- und Diglyceride von Fettsäuren, Natriumcaprylat, Natriumlaurylethersulfat, Na-/K-Salze von Fettsäuren, Na-Laurylsulfat, PEG-(2)-stearat, Natriumsulfosuccinat, Polyglycerylester von Fettsäuren, Polyoxyethylenalkylether, Cetomacrogol, Polyoxyethylen-Fettsäure-Sorbitanester, Propylenglykolstearat, Sorbitanfettsäureester, Stearinsäurediethanolamid, mittelkettige (vorzugsweise C₁₂-C₁₄) Triglyceride (nach Ph. Eur.).

Als Lösungsvermittler können N-Methyl-2-pyrrolidon, Laurylpyrrolidon, Triethanolamin, Triacetin, Diethylenglykol-monoethylether, Derivate von Fettsäuren oder Fettalkoholen und/oder niedermolekulare, mehrwertige Alkohole wie beispielsweise Propylenglykol oder Glycerin verwendet werden.

Bei einer viskositätserhöhenden Substanz im Sinne der Beschreibung handelt es sich bevorzugt um einen Gelbildner, d.h. um eine Substanz, welche einerseits die Viskosität der Zusammensetzung erhöht, andererseits deren Gelbildung unterstützt. Beispiele für solche viskositätserhöhende Substanzen sind Gelatine (z.B. Gelatin), Johannisbrotkernmehl (z.B. Cesagum® LA-200, Cesagum® LID/150, Cesagum® LN-1), Pektine wie Citrus-Pectin (z.B. Cesapectin® HM Medium Rapid Set), Apfelpektin, Pektin aus Zitronenschale, Wachsmaisstärke (C*Gel® 04201), Natriumalginat (z.B. Frimulsion® ALG (E401)), Guarkernmehl (z.B. Frimulsion® BM, Polygum® 26/1-75), lota-Carrageen (z.B. Frimulsion® D021), Karaya Gummi, Gellangummi (z.B. Kelcogel® F, Kelcogel® LT100), Galaktomannan (z.B. Meyprogat® 150), Tarakernmehl (z.B. Polygum® 43/1), Propylenglykolalginat (z.B. Protanal®-Ester SD-LB), Natrium-Hyaluronat, Tragant, Taragummi (z.B. Vidogum® SP 200), fermentiertes Polysaccharid-Welan Gum (z.B. K1A96), Xanthan-Gummi (z.B. Xantural® 180).

Wenn das erfindungsgemäße TTS nach dem Reservoir-System aufgebaut ist, kann die Reservoir-Membran aus inerten Polymeren, wie z.B. Polyethylenen, Polypropylenen, Polyvinylacetaten, Polyamiden, Ethylen/Vinylacetat Copolymeren und/oder Silikonen bestehen. Durch die Reservoir-Membran kann aus dem Reservoir eine kontrollierte Freisetzung des Wirkstoffes erzielt werden.

Die wirkstoffhaltige Matrix bzw. das wirkstoffhaltige Reservoir kann auch ein Lösungsmittel, wie z.B. Wasser, Ethanol, 1-Propanol, Isopropanol, einen niedermolekularen, mehrwertigen Alkohol, beispielsweise Propylenglykol oder Glycerin, oder einen Ester, wie Isopropylmyristat, oder deren Mischungen enthalten. In einer bevorzugten Ausführungsform enthält die wirkstoffhaltige Matrix bzw. das wirkstoffhaltige Reservoir jedoch im Wesentlichen kein Ethanol und/oder kein Isopropanol.

Als Konservierungsmittel für die wirkstoffhaltige Schicht können Antioxidantien, wie Vitamin E, Butylhydroxytoluol, Butylhydroxyanisol, Parabene, Ascorbinsäure, Ascorbylpalmitat, und/oder Chelatbildner, wie Dinatriumethylendiamintetraessigsäure, Kalium- oder Natriumcitrat verwendet werden. Als Konservierungsmittel eignen sich ferner PHB-Ester, wie PHB-Methylester und PHB-Propylester, Benzalkoniumchlorid, Chlorhexidin, Sorbinsäure, Benzoesäure, Propionsäure, Salicylsäure und deren Salze, Formaldehyd und Paraformaldehyd, Hexachlorophen, o-Phenylphenol, Zinkpyrithion, anorganische Sulfite, Natriumiodat, Chlorbutol, Dehydroessigsäure, Ameisensäure, Dibromhexamid, Merthiolat, Phenylmercurisalze (Phenyl-Hg-Verbindungen), Undecylensäure, Hexetidine, Bronidox, Bronopol, 2,4-Dichlorbenzylalkohol, Triclocarban, Parachlormetakresol, Triclosan, Parachlormetaxylenol, Imidazolydinyl-Harnstoff-Derivate, Polyhexamethylenbiguanidochorhydrat, Phenoxyethanol, Methenamin, Dowicil 200, 1-Imidazolyl-(4-chlorphenoxy)-3,3'-dimethylbutan-2-on, Dimethylhydantoin, Benzylalkohol, 1-Hydroxy-4-methyl-6-(2,4,4-trimethyl-pentyl)-2-pyridon, 1,2-Dibrom-2,4-dicyanobutan, 3,3'-Dibrom-5,5'-dichlor-2,2-dihydroxydiphenylmethan, Isopropylmetakresol und Kathon.

Das erfindungsgemäße TTS kann in einer Schicht oder in mehreren Schichten auch wenigstens einen Weichmacher ausgewählt aus der Gruppe umfassend langkettige Alkohole wie Dodecanol, Undecanol, Octanol, Ester von Carbonsäuren mit polyethoxylierten Alkoholen, Diester von aliphatischen Dicarbonsäuren wie Adipinsäure, und mittelkettige Triglyceride von Caprylsäure und/oder Caprinsäure, Kokosfett, mehrwertige Alkohole wie 1,2-Propandiol, Ester von mehrwertigen Alkoholen wie Glycerin mit Lävulinsäure oder Caprylsäure, und veretherte mehrwertige Alkohole enthalten.

Die Herstellung des erfindungsgemäßen TTS kann nach den bekannten Herstellungsverfahren, insbesondere für wirkstoffhaltige Pflaster mit Verfahrensschritten wie Laminieren, Stanzen, Delaminieren, Abwickeln, Schneiden, Wiederaufwickeln, Montieren oder Dosieren erfolgen (vgl. Verpackungs-Rundschau 4/2002, 83-84).

Die Wirkstofffreisetzung aus dem erfindungsgemäßen TTS erfolgt bevorzugt kontrolliert. Dabei weist das erfindungsgemäße TTS bevorzugt eine maximale Freisetzungsrate des Wirkstoffs von höchstens 100 µg/cm² h, bevorzugt höchstens 95 µg/cm² h, höchstens 90 µg/cm² h oder höchstens 85 µg/cm² h; bevorzugter höchstens 80 µg/cm² h, höchstens 75 µg/cm² h oder höchstens 70 µg/cm² h; noch bevorzugter höchstens 65 µg/cm² h, höchstens 60 µg/cm² h, oder höchstens 55 µg/cm² h; am bevorzugtesten höchstens 50 µg/cm² h, höchstens 45 µg/cm² h oder höchstens 40 µg/cm² h; und insbesondere höchstens 35 µg/cm² h oder höchstens 32 µg/cm² h.

In einer bevorzugten Ausführungsform liegt die maximale Freisetzungsrate des Wirkstoffs im Bereich von 20±15 µg/cm² h, bevorzugter 20±12,5 µg/cm² h, noch bevorzugter 20±10 µg/cm² h, am bevorzugtesten 20±7,5 µg/cm² h und insbesondere 20±5 µg/cm² h. In einer anderen bevorzugten Ausführungsform liegt die maximale Freisetzungsrate des Wirkstoffs im Bereich von 25±10 µg/cm² h, noch bevorzugter 25±7,5 µg/cm² h, am bevorzugtesten 25±5 µg/cm² h und insbesondere 25±2,5 µg/cm² h. In einer anderen bevorzugten Ausführungsform liegt die maximale Freisetzungsrate des Wirkstoffs im Bereich von 30±5 µg/cm² h, noch bevorzugter 30±2,5 µg/cm² h.

Üblicherweise verändert sich die Freisetzungsrate während des Applikationsintervalls des TTS. So nimmt einerseits die Wirkstoffmenge im TTS infolge der Freisetzung kontinuierlich ab, andererseits kann die Haut eine gewisse Speicherwirkung entfalten. Beide Effekte können die Freisetzungsrate beeinflussen.

Die maximale Freisetzungsrate eines TTS und ihre Bestimmung sind dem Fachmann bekannt. Die maximale Freisetzungsrate kann als maximale humane Hautpermeation angegeben und *in vitro* beispielsweise mit Hilfe einer Franz-Zelle bestimmt werden. Vorzugsweise erfolgt die Bestimmung unter den im experimentellen Teil ausgeführten Bedingungen.

Das erfindungsgemäße TTS weist eine tₘₐₓ von mindestens 24 h, bevorzugter mindestens 26 h, noch bevorzugter mindestens 28 h, am bevorzugtesten mindestens 30 h und insbesondere mindestens 32 h auf. In einer bevorzugten Ausführungsform des erfindungsgemäßen TTS liegt tₘₐₓ im Bereich von 36±12 h, bevorzugter 36±10 h, noch bevorzugter 36±8 h, am bevorzugtesten 36±6 h, und insbesondere 36±4 h. In einer anderen bevorzugten Ausführungsform des erfindungsgemäßen TTS liegt tₘₐₓ im Bereich von 32±8 h, bevorzugter 32±7 h, noch bevorzugter 32±6 h, am bevorzugtesten 32±5 h, und insbesondere 32±4 h. In einer weiteren bevorzugten Ausführungsform des erfindungsgemäßen TTS liegt tₘₐₓ im Bereich von 28±4 h, bevorzugter 28±3 h, am bevorzugtesten 28±2 h.

Dabei ist tₘₐₓ vorzugsweise als diejenige Zeit definiert, nach welcher während der einmaligen und erstmaligen Applikation des erfindungsgemäßen TTS die maximale Wirkstoffkonzentration im Blutplasma (Cₘₐₓ) erreicht wird, gemessen ab dem Aufbringen des TTS auf der Haut. Geeignete Methoden zur Bestimmung von tₘₐₓ und Cₘₐₓ sind dem Fachmann bekannt.

Das erfindungsgemäße TTS weist bevorzugt eine Cₘₐₓ von mindestens 4 ng/mL, mindestens 4,5 ng/mL, mindestens 5 ng/mL oder mindestens 5,5 ng/mL auf, bevorzugter mindestens 6 ng/mL, noch bevorzugter mindestens 8 ng/mL, am bevorzugtesten mindestens 10 ng/mL und insbesondere mindestens 12 ng/mL.

Das erfindungsgemäße TTS weist bevorzugt eine pharmakokinetische Verzögerungszeit t_{lag} von höchstens 6 h auf, bevorzugter 1,33±1,00 h, noch bevorzugter 1,33±0,66 h und insbesondere 1,33±0,33 h. Die pharmakokinetische Verzögerungszeit t_{lag} des erfindungsgemäßen TTS ist damit überraschenderweise kurz, was Vorteile mit sich bringt. So werden wirksame Plasmakonzentrationen des Wirkstoffs in vergleichsweise kurzer Zeit nach dem Aufbringen des TTS auf der Haut erreicht.

Das erfindungsgemäße TTS weist bevorzugt eine Dauer der bioanalytischen Nachweisbarkeit tₗₐₛₜ von mindestens 200 h auf, bevorzugter mindestens 220 h und insbesondere mindestens 240 h.

Das erfindungsgemäße TTS weist bevorzugt eine pharmakokinetische Halbwertszeit des Wirkstoffs t_{1/2,z} im Bereich von 60-100 h auf, bevorzugter im Bereich von 52±25 h, bevorzugter 52±15 h, am bevorzugtesten 52±10 h und insbesondere 52±5 h auf.

Geeignete Methoden zur Bestimmung dieser pharmakokinetischen Parameter sind dem Fachmann bekannt. Die Bestimmung der Wirkstoffkonzentration im Blutplasma ist dem Fachmann ebenfalls bekannt. Sie erfolgt vorzugsweise durch LC, beispielsweise HPLC, vorzugsweise gekoppelt mit MS (LC-MS bzw. HPLC-MS).

Das erfindungsgemäße TTS weist vorzugsweise eine geringe interindividuelle Variabilität auf, bevorzugt höchstens 25%, bevorzugter höchstens 20%. Eine derart geringe interindividuelle Variabilität hat den Vorteil, dass die *in vivo* erreichten Blutspiegel gut vorhergesagt werden können, was u.a. auch mit einer erhöhten therapeutischen Sicherheit einhergeht.

Mit höchstens 25% interindividueller Variabilität der pharmakokinetischen Parameter unterscheidet sich das erfindungsgemäße TTS von anderen TTS, welche zur verabreichung anderer Wirkstoffe vorgesehen sind. Beispielsweise wurde über ein TTS zur Verabreichung des Wirkstoffs Rivastigmin berichtet, bei welchem die Variabilität der Parameter bei >32% liegt (vgl. G. Lefèvre et al., Clinical Pharmacology & Therapeutics, 83(1), 2008, 106-114).

In einer bevorzugten Ausführungsform weist das erfindungsgemäße TTS nach bestimmungsgemäßer Anwendung einen vergleichsweise geringen Restgehalt an Wirkstoff auf. Vorzugsweise beträgt der Restgehalt nach 24 h Applikation höchstens 75 Gew.-%, bevorzugter höchstens 70 Gew.-%, noch bevorzugter höchstens 65 Gew.-% und insbesondere höchstens 60 Gew.-% der ursprünglich im TTS enthaltenen Menge. Dies ist aus ökonomischen Gründen und auch aus Sicherheitsgründen von Vorteil.

Das erfindungsgemäße TTS ist derart ausgestaltet, dass bei aufeinanderfolgender, oder zeitlich versetzter, bevorzugt wiederholter, Applikation jeweils eines oder mehrerer der erfindungsgemäßen TTS für Applikationsintervalle von jeweils unabhängig voneinander mindestens 12 h über einen Gesamtzeitraum von mindestens 300 h am behandlungsbedürftigen Individuum in dessen Blutplasma jeweils ab Beginn der erstmaligen Applikation folgende Wirkstoffkonzentrationen erreicht werden:
nach 24 h mindestens 14 ng/ml, bevorzugt mindestens 16,2 ng/mL,
nach 48 h mindestens 27 ng/ml, bevorzugt mindestens 29,2 ng/mL,
nach 60 h mindestens 32 ng/ml, bevorzugt mindestens 34,5 ng/mL,
nach 72 h mindestens 37 ng/ml, bevorzugt mindestens 39,1 ng/mL,
nach 96 h mindestens 44 ng/ml, bevorzugt mindestens 46,6 ng/mL,
nach 144 h mindestens 54 ng/ml, bevorzugt mindestens 56,5 ng/mL,
nach 192 h mindestens 60 ng/ml, bevorzugt mindestens 62,3 ng/mL,
nach 240 h mindestens 63 ng/ml, bevorzugt mindestens 65,5 ng/mL, und
nach 300 h mindestens 65 ng/ml, bevorzugt mindestens 67,8 ng/mL.

Dabei erfolgt die wiederholte Applikation unmittelbar aufeinander, d.h. wenn das oder die erfindungsgemäßen TTS am Ende eines Applikationsintervalls entfernt werden, werden unverzüglich das oder die erfindungsgemäßen TTS für das sich daran anschließende Applikationsintervall appliziert. Dabei beträgt das Zeitintervall, zu dem am Körper ggf. keinerlei erfindungsgemäße TTS angebracht sind, höchstens 10 Minuten, bevorzugter höchstens 5 Minuten.

In einer bevorzugten Ausführungsform erfolgt die Applikation aller erfindungsgemäßen TTS über den Gesamtzeitraum auf derselben Hautpartie des Individuums, d.h. eine gegebene Hautpartie des Individuums wird wiederholt mit erfindungsgemäßen TTS belegt bzw. beklebt.

In einer anderen bevorzugten Ausführungsform erfolgt die Applikation aller erfindungsgemäßen TTS über den Gesamtzeitraum auf jeweils anderen Hautpartien des Individuums, d.h. eine gegebene Hautpartie des Individuums wird nicht wiederholt mit erfindungsgemäßen TTS belegt bzw. beklebt.

Dabei werden die erfindungsgemäßen TTS für aufeinanderfolgende Applikationsintervalle von jeweils unabhängig voneinander mindestens 12 h, vorzugsweise über einen Gesamtzeitraum von mindestens 300 h, appliziert. Dauert jedes Applikationsintervall z.B. 12 h, so sind wenigstens 25 aufeinanderfolgende Applikationsintervalle erforderlich, um den Gesamtzeitraum von mindestens 300 h abzudecken. Dauert jedes Applikationsintervall z.B. 18 h, so sind entsprechend wenigstens 17 aufeinanderfolgende Applikationsintervalle erforderlich, um den Gesamtzeitraum von mindestens 300 h abzudecken.

Die einzelnen Applikationsintervalle können dieselbe oder unterschiedliche Länge haben. Vorzugsweise sind alle Applikationsintervalle gleichlang, bevorzugt jeweils 12 h, 14 h, 16 h, 18 h, 20 h, 22 h, 24 h, 26 h, 28 h, 30 h, 32 h, 34 h, 36 h, 38 h, 40 h, 42 h, 44 h, 46 h, 48 h, 50 h, 52 h, 54 h, 56 h, 58 h, 60 h, 62 h, 64 h, 66 h, 68 h, 70 h oder 72 h. In einer bevorzugten Ausführungsform beträgt jedes Applikationsintervall unabhängig voneinander höchstens 168 h oder höchstens 144 h, bevorzugter höchstens 120 h oder höchstens 96 h, noch bevorzugter höchstens 72 h oder höchstens 48 h, am bevorzugtesten höchstens 36 h oder höchstens 24 h und insbesondere höchstens 12 h.

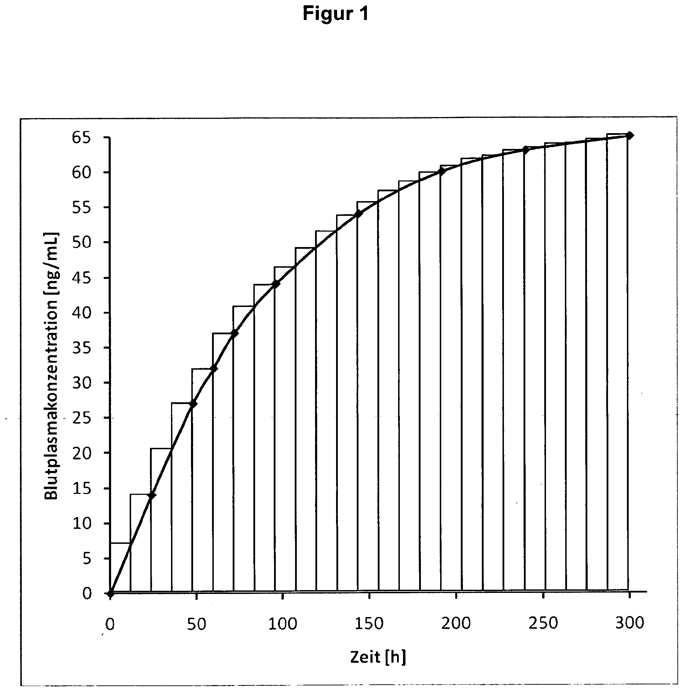

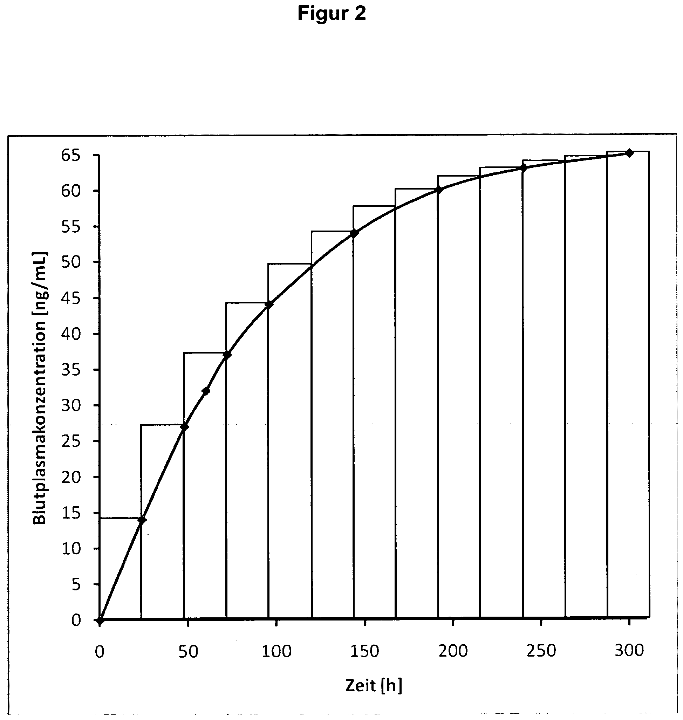

Die Applikationsintervalle sind in Figuren 1 und 2 näher erläutert. Beide Figuren zeigen schematisch den Verlauf der minimalen Blutplasmakonzentration des Wirkstoffs über die Zeit. In Figur 1 steht jede Säule im Diagramm für ein Applikationsintervall von 12 h, in Figur 2 für ein Applikationsintervall von 24 h. Die Höhe der Säulen steht jeweils für die minimal erreichte Blutplasmakonzentration.

Während jedes Applikationsintervalls kann ein einziges erfindungsgemäßes TTS appliziert werden oder es können jeweils gleichzeitig mehrere erfindungsgemäße TTS appliziert werden. Die Anzahl der applizierten TTS kann von Applikationsintervall zu Applikationsintervall verschieden sein. Vorzugsweise ist die Anzahl der applizierten TTS in jedem Applikationsintervall identisch.

In einer bevorzugten Ausführungsform werden die genannten Wirkstoffkonzentrationen bei Applikation von jeweils einem oder von gleichzeitig zwei, drei, vier, fünf, sechs, sieben, acht, neun, zehn, elf, zwölf, dreizehn, vierzehn, fünfzehn oder sechszehn der erfindungsgemäßen TTS pro Applikationsintervall erreicht.

In einer bevorzugten Ausführungsform liegt für jedes Applikationsintervall die Dauer in h dividiert durch die Anzahl der ggf. gleichzeitig applizierten TTS unabhängig voneinander im Bereich von 4 bis 24, bevorzugter 5 bis 20, noch bevorzugter 6 bis 18. In einer bevorzugten Ausführungsform liegt für jedes Applikationsintervall die Dauer in h dividiert durch die Anzahl der ggf. gleichzeitig applizierten TTS unabhängig voneinander im Bereich von 3 bis 8, bevorzugter 4 bis 7 und insbesondere ca. 6.

Bevorzugte Ausführungsformen des erfindungsgemäßen TTS, welche die vorstehend beschriebenen Wirkstoffkonzentrationen erreichen, sind nachfolgend aufgelistet: 12², 12³, 12⁴, 18², 18³, 18⁴, 18⁵, 24³, 24⁴, 24⁵, 24⁶, 24⁷, 36⁵, 36⁶, 36⁷, 36⁸, 36⁹, 36¹⁰, 36¹¹, 48⁷, 48⁸, 48⁹, 48¹⁰, 48¹¹, 48¹², 48¹³, 48¹⁴, 48¹⁵, 48¹⁶, 60⁹, 60¹⁰, 60¹¹, 60¹², 60¹³, 60¹⁴, 60¹⁵, 60¹⁶, 60¹⁷, 60¹⁸, 60¹⁹, 72¹², 72¹³, 72¹⁴, 72¹⁵, 72¹⁶, 72¹⁷, 72¹⁸, 72¹⁹, 72²⁰, 72²¹, 72²², 84¹⁴, 84¹⁵, 84¹⁶, 84¹⁷, 84¹⁸, 84¹⁹, 84²⁰, 84²¹, 84²², 84²³, 84²⁴, 96¹⁹, 96²⁰, 96²¹, 96²², 96²³, 96²⁴. Dabei ist zunächst die Dauer jedes Applikationsintervalls in Stunden angegeben (z.B. "12") und danach hochgestellt die Anzahl der jeweils ggf. gleichzeitig applizierten erfindungsgemäßen TTS (z.B. "³"). Dementsprechend bedeutet z.B. "24²" die jeweils gleichzeitige Applikation zweier erfindungsgemäßer TTS für aufeinanderfolgende Applikationsintervalle von jeweils 24 h.

Bevorzugt ist das erfindungsgemäße TTS dafür hergerichtet, gemäß den vorstehend definierten Applikationsintervallen angewendet zu werden, dies zur Behandlung von oder Vorbeugung gegen Alzheimer umfassend die aufeinanderfolgende Verabreichung mehrerer erfindungsgemäßer TTS unter Einhaltung der vorstehend definierten Applikationsintervalle.

Ein Fachmann erkennt, dass ein bestimmtes Verhältnis von Applikationsdauer zu Anzahl der ggf. gleichzeitig zu applizierenden TTS bei Verringerung der Anzahl der gleichzeitig zu applizierenden TTS gleichwirkend durch eine entsprechende Vergrößerung des TTS verwirklicht werden kann und umgekehrt. So ist die pharmakologische Wirkung eines TTS mit einer wirkstoffabgebenden Fläche von z.B. 5 cm² näherungsweise doppelt so hoch wie die pharmakologische Wirkung eines ansonsten identischen TTS mit einer wirkstoffabgebenden Fläche von 2,5 cm².

Die wirkstoffabgebende Fläche (wirkstoffhaltige Kontaktfläche) des erfindungsgemäßen TTS liegt vorzugsweise im Bereich von 0,1 bis 400 cm². In einer bevorzugten Ausführungsform liegt die wirkstoffabgebende Fläche des erfindungsgemäßen TTS im Bereich von 10±9 cm², bevorzugter 10±7,5 cm², noch bevorzugter 10±5 cm², am bevorzugtesten 10±2,5 cm² und insbesondere 10±1 cm². In einer anderen bevorzugten Ausführungsform liegt die wirkstoffabgebende Fläche des erfindungsgemäßen TTS im Bereich von 20±9 cm², bevorzugter 20±7,5 cm², noch bevorzugter 20±5 cm², am bevorzugtesten 20±2,5 cm² und insbesondere 20±1 cm². In einer anderen bevorzugten Ausführungsform liegt die wirkstoffabgebende Fläche des erfindungsgemäßen TTS im Bereich von 25±20 cm², bevorzugter 25±15 cm², noch bevorzugter 25±10 cm², am bevorzugtesten 25±5 cm² und, insbesondere 25±2,5 cm². In einer weiteren bevorzugten Ausführungsform liegt die wirkstoffabgebende Fläche des erfindungsgemäßen TTS im Bereich von 30±9 cm², bevorzugter 30±7,5 cm², noch bevorzugter 30±5 cm², am bevorzugtesten 30±2,5 cm² und insbesondere 30±1 cm². In einer anderen bevorzugten Ausführungsform liegt die wirkstoffabgebende Fläche des erfindungsgemäßen TTS im Bereich von 100±90 cm², bevorzugter 100±75 cm², noch bevorzugter 100±50 cm², am bevorzugtesten 100±25 cm² und insbesondere 100±10 cm². In einer weiteren bevorzugten Ausführungsform liegt die wirkstoffabgebende Fläche des erfindungsgemäßen TTS im Bereich von 200±90 cm², bevorzugter 200±75 cm², noch bevorzugter 200±50 cm², am bevorzugtesten 200±25 cm² und insbesondere 200±10 cm².

Ist die wirkstoffabgebende Fläche groß genug, z.B. größer als beispielsweise 10 cm², so kann durch Abdecken eines Teils der wirkstoffhaltigen Fläche mit Hilfe einer wirkstoffundurchlässigen Schutzschicht die tatsächliche wirkstoffabgebende Fläche verringert werden. Weist das erfindungsgemäße TTS beispielsweise eine wirkstoffhaltige, quadratische Fläche von 6 x 6 cm auf, so sind 26 cm² dieser Fläche mit einer wirkstoffundurchlässigen Schicht zu bedecken, um eine wirkstoffabgebende Fläche von 10 cm² zu gewährleisten. Ein Fachmann erkennt, dass ggf. stattfindende Querdiffusion zu berücksichtigen ist, insbesondere bei Substanzen, welche gut in die klebstoffhaltige Schicht permeieren, so dass die Größe der Schutzschicht ggf. angepasst werden muss.

In einer bevorzugten Ausführungsform des erfindungsgemäßen TTS werden jeweils ab Beginn der erstmaligen Applikation folgende Wirkstoffkonzentrationen erreicht:
nach 24 h höchstens 50 ng/ml,
nach 48 h höchstens 90 ng/ml,
nach 60 h höchstens 110 ng/ml,
nach 72 h höchstens 130 ng/ml,
nach 96 h höchstens 150 ng/ml,
nach 144 h höchstens 180 ng/ml,
nach 192 h höchstens 195 ng/ml,
nach 240 h höchstens 200 ng/ml, und
nach 300 h höchstens 210 ng/ml.

Bevorzugte Bereiche B₁ bis B₄ der Wirkstoffkonzentration sind in nachfolgender Tabelle zusammengefasst:

| nach | B1 | B2 | B3 | B4 |
|---|---|---|---|---|
| 24 h | 24,3 ng/mL ± 30% | 22,7 ng/mL ± 25% | 21,1 ng/mL ± 20% | 20,3 ng/mL ± 15% |
| 48 h | 43,8 ng/mL ± 30% | 40,9 ng/mL ± 25% | 38,0 ng/mL ± 20% | 36,5 ng/mL ± 15% |
| 60 h | 51,8 ng/mL ± 30% | 48,3 ng/mL ± 25% | 44,9 ng/mL ± 20% | 43,1 ng/mL ± 15% |
| 72 h | 58,7 ng/mL ± 30% | 54,7 ng/mL ± 25% | 50,8 ng/mL ± 20% | 48,9 ng/mL ± 15% |
| 96 h | 69,9 ng/mL ± 30% | 65,2 ng/mL ± 25% | 60,6 ng/mL ± 20% | 58,3 ng/mL ± 15% |
| 144 h | 84,9 ng/mL ± 30% | 79,2 ng/mL ± 25% | 73,6 ng/mL ± 20% | 70,8 ng/mL ± 15% |
| 192 h | 93,5 ng/mL ± 30% | 87,2 ng/mL ± 25% | 81,0 ng/mL ± 20% | 77,9 ng/mL ± 15% |
| 240 h | 98,3 ng/mL ± 30% | 91,7 ng/mL ± 25% | 85,2 ng/mL ± 20% | 81,9 ng/mL ± 15% |
| 300 h | 101,7 ng/mL ± 30% | 94,9 ng/mL ± 25% | 88,1 ng/mL ± 20% | 84,8 ng/mL ± 15% |

In einer bevorzugten Ausführungsform werden bei kontinuierlicher, bestimmungsgemäßer Anwendung der erfindungsgemäßen TTS Steady-State-Plamakonzentrationen des Wirkstoffs im Bereich von 70 bis 150 ng/mL erreicht.

In einer bevorzugten Ausführungsform des erfindungsgemäßen TTS liegt seine über das Applikationsintervall gemittelte Freisetzungsrate des Wirkstoffs im Bereich von 0,2 bis 2,0 mg/h. In einer bevorzugten Ausführungsform liegt diese gemittelte Freisetzungsrate im Bereich von 0,7±0,5 mg/h, bevorzugter 0,7±0,4 mg/h, noch bevorzugter 0,7±0,3 mg/h. In einer anderen bevorzugten Ausführungsform liegt diese gemittelte Freisetzungsrate im Bereich von 1,0±0,5 mg/h, bevorzugter 1,0±0,4 mg/h, noch bevorzugter 1,0±0,3 mg/h. In einer weiteren bevorzugten Ausführungsform liegt diese gemittelte Freisetzungsrate im Bereich von 1,3±0,5 mg/h, bevorzugter 1,3±0,4 mg/h, noch bevorzugter 1,3±0,3 mg/h. In einer anderen bevorzugten Ausführungsform liegt diese gemittelte Freisetzungsrate im Bereich von 1,5±0,5 mg/h, bevorzugter 1,5±0,4 mg/h, noch bevorzugter 1,5±0,3 mg/h.

Bevorzugt entspricht die in einem erfindungsgemäßen TTS enthaltene, transdermal freisetzbare Wirkstoffmenge mindestens einer halben Tagesdosis, vorzugsweise mindestens einer ganzen Tagesdosis, welche zur Behandlung bzw. Vorbeugung der betreffenden Erkrankung des Zentralnervensystems erforderlich ist. Die Höhe der erforderlichen Tagesdosis ist dem Fachmann bekannt.

In einer bevorzugten Ausführungsform enthält das erfindungsgemäße TTS die entsprechende Dosis für mindestens
- 1 Tag (transdermal freisetzbare Dosis = einfache Tagesdosis),
- 2 Tage (transdermal freisetzbare Dosis = doppelte Tagesdosis),
- 3 Tage (transdermal freisetzbare Dosis = dreifache Tagesdosis),
- 4 Tage (transdermal freisetzbare Dosis = vierfache Tagesdosis),
- 5 Tage (transdermal freisetzbare Dosis = fünffache Tagesdosis),
- 6 Tage (transdermal freisetzbare Dosis = sechsfache Tagesdosis) oder
- 7 Tage (transdermal freisetzbare Dosis = siebenfache Tagesdosis).

Handelt es sich bei der Erkrankung um mittel-schwere bis schwere Alzheimer-Krankheit, so liegt die bevorzugte tägliche, transdermal verabreichte Dosis bei ca. 20 mg (Äquivalentmenge bezogen auf Memantin-Hydrochlorid). In den ersten Wochen der Behandlung kann es jedoch empfehlenswert sein, mit einer geringeren täglichen, transdermal verabreichten Dosis zu beginnen und diese mit der Zeit zu steigern, bis ein Wert von etwa 20 mg erreicht ist. Beispielsweise ist es bevorzugt, dass die tägliche, transdermal verabreichte Dosis in der ersten Woche etwa 5,0 mg, in der zweiten Woche etwa 10 mg, in der dritten Woche etwa 15 mg und danach kontinuierlich etwa 20 mg beträgt (Äquivalentmenge jeweils bezogen auf Memantin Hydrochlorid). Bevorzugt beträgt die Flächenkonzentration von Memantin bzw. eines seiner physiologisch verträglichen Salze mindestens 1,0 mg/cm², bevorzugter mindestens 5,0, 10 oder 15 mg/cm², noch bevorzugter mindestens 20, 25 oder 30 mg/cm², am bevorzugtesten mindestens 35, 40 oder 50 mg/cm² und insbesondere mindestens 60 oder 70 mg/cm².

In einer bevorzugten Ausführungsform weist das erfindungsgemäße TTS eine Gesamtschichtdicke im Bereich von 50 bis 4.000 µm auf, bevorzugter 100 bis 2.500 µm, noch bevorzugter 200 bis 2.000 µm, am bevorzugtesten 250 bis 1.500 µm und insbesondere 300 bis 1.200 µm.

Ein weiterer illustrativer Aspekt der Erfindung betrifft eine Packungseinheit umfassend wenigstens zwei verschiedene Typen der vorstehend beschriebenen erfindungsgemäßen TTS, welche sich vorzugsweise in der darin enthaltenen, täglich freisetzbaren Dosis (Memantin bzw. eines seiner physiologisch verträglichen Salze), unterscheiden. Bevorzugt sind wenigstens 2, bevorzugter wenigstens 3 und insbesondere wenigstens 4 verschiedene TTS-Typen mit unterschiedlicher täglich freisetzbarer Dosis vorhanden, wobei TTS-Typen mit derselben täglich freisetzbaren Dosis vorzugsweise mehrfach vertreten sein können (mehrere Typen derselben Art).

In einer bevorzugten Ausführungsform umfasst die erfindungsgemäße Packungseinheit so viele TTS wie erforderlich sind, um ausgehend von einer täglich freisetzbaren Anfangsdosis durch schrittweise Steigerung (Titration) eine täglich freisetzbare Maximaldosis zu erreichen. Vorzugsweise wird diese Maximaldosis über 2, 3 oder 4 Stufen erreicht, wobei jede Stufe gleiche oder verschiedene Zeiträume, einzelne oder mehrere Anwendungsintervalle, umfassen kann. Ein Anwendungsintervall ist in diesem Zusammenhang derjenige Zeitraum, für welchen bei bestimmungsgemäßer Anwendung des betreffenden TTS die avisierte tägliche, transdermal zu verabreichende Dosis tatsächlich freisetzbar ist. Umfasst ein Anwendungsintervall beispielsweise 48 h, so muss das TTS gewährleisten, dass über diesen Zeitraum die betreffende tägliche, transdermal zu verabreichende Dosis tatsächlich aus dem TTS freisetzbar ist.

In einer bevorzugten Ausführungsform entsprechen die Anwendungsintervalle den vorstehend im Zusammenhang mit der Wirkstoffkonzentration beschriebenen Applikationsintervallen.

Die nachfolgenden Beispiele dienen zur Erläuterung der Erfindung, sind jedoch nicht einschränkend auszulegen:

### Beispiel 1:

Es wurde ein TTS in Form eines wirkstoffhaltigen Pflasters folgender Zusammensetzung hergestellt:

| | |
|---|---|
| Memantin Base | 6,36 Gew.-% |
| Triglycerid mittlerer Kettenlänge (z.B. Miglyol 812) | 25,42 Gew.-% |
| Acrylat-Vinylacetat Copolymer (z.B. Durotak 2516) | 68,22 Gew.-% |
| wirkstoffhaltige Fläche | 10 cm² |
| Wirkstoffgehalt (berechnet als Base) | 11,0 mg |

Dazu wurde eine Dosierlösung aus Memantin (freie Base) und Miglyol 812 mit folgendem Mischungsverhältnis hergestellt.

| | |
|---|---|
| Memantin Base | 20,00 Gew.-% |
| Triglycerid mittlerer Kettenlänge (z.B. Miglyol 812) | 80,00 Gew.-% |

Das entsprechende Acrylat z.B. Durotak® 2516 wird auf eine geeignete Schutzfolie (z.B. Polyethylentherephtalat-Folie, PET) beschichtet, getrocknet und eine geeignete okklusive Trägerschicht zukaschiert (1. Schicht). Anschließend wird eine 2. Schicht aus z.B. Durotak® 2516 wie oben beschrieben hergestellt, aber nicht mit einer geeigneten Trägerschicht zukaschiert. Stattdessen wird ein geeignetes Gewebe z.B. Langfaserpapier LF26 definierter Größe (z.B. 10 cm²) auf die getrocknete Acrylatschicht aufgelegt und eine entsprechende Menge der Dosierlösung auf das geeignete Gewebe dosiert. Die 1. Schicht wird auf die das Gewebe enthaltende 2. Schicht nach Entfernen der Schutzfolie zukaschiert. Nach Stanzen der TTS (in der Form, dass das Gewebe mittig liegt) werden diese in einem geeigneten Material verpackt, bevorzugt PET.

### Beispiel 2:

In Anlehnung an Beispiele 1 wurden acht verschiedene TTS in Form wirkstoffhaltiger Pflaster (drug in adhesive) folgender Zusammensetzung hergestellt:

| Gew.-% | | | | | | | | |
|---|---|---|---|---|---|---|---|---|
| | 2-1 | 2-2 | 2-3 | 2-4 | 2-5 | 2-6 | 2-7 | 2-8 |
| Memantin Base | 6,00 | 6,00 | - | - | - | - | - | - |
| Memantin HCl | - | - | 40,00 | 10,00 | 30,00 | 30,00 | 30,00 | 30,00 |
| CaO | - | - | - | - | 7,50 | 7,50 | 7,50 | 7,50 |
| Na-Caprylat | - | - | 10,00 | - | - | - | - | - |
| Na-Ethanolat | - | - | - | 7,00 | - | - | - | - |
| basisches Methacrylat-Copolymer (z.B. Eudragit® E 100) | - | - | 20,00 | - | - | - | - | - |
| Poly(butylmethacrylat, methylmethacrylat) (z.B. Plastoid® B) | 2,00 | 2,00 | - | - | - | - | - | - |
| Acrylat-Vinylacetat Copolymer (z.B. Durotak® 2516) | 92,00 | - | - | 83,00 | - | - | - | - |
| Acrylat-Vinylacetat Copolymer (z.B. Durotak® 2287) | - | 92,00 | 30,00 | - | - | - | - | - |
| Polyisobutylen | - | - | - | - | 62,50 | - | - | - |
| neutrales Polyacrylat (z.B. Durotak® 900A) | - | - | - | - | - | 62,50 | - | - |
| neutrales Polyacrylat (z.B. Durotak® 9301) | - | - | - | - | - | - | 62,50 | - |
| neutrales Polyacrylat (z.B. GMS 3083) | | | - | - | - | - | - | 62,50 |
| wirkstoffhaltige Fläche | 30 cm² | 30 cm² | 30 cm² | 30 cm² | 10 cm² | 10 cm² | 10 cm² | 10 cm² |
| Wirkstoffgehalt (berechnet als Base) | 39,8 mg | 48,9 mg | 98,2 mg | 27,5 mg | 24,7 mg | 23,4 mg | 25,6 mg | 24,8 mg |

Die entsprechende Menge an Memantin-hydrochlorid bzw. -base wird vorgelegt und in ca. 1/3 der Menge der Menge an Wirkstoff mit einem organischen Lösungsmittel (z.B. Ethylacetat) angeschlemmt bzw. für die Base gelöst. Nach Zugabe der entsprechenden Menge eines geeigneten Acrylates wird gerührt, um eine homogene Verteilung des Wirkstoffs im Polymer zu gewährleisten.

Anschließend werden die entsprechenden weiteren Rohstoffe (Plastoid® B für 2-1 und 2-2 bzw. Na-Caprylat und Eudragit® E100 für 2-3 bzw. Na-Ethanolat für 2-4 bzw. CaO für 2-5 bis 2-8) unter Rühren in die Masse gegeben und weitergerührt, bis eine homogene Masse entsteht. Nach Beschichten auf eine geeignete Schutzfolie (z.B. Polyethylentherephtalat-Folie, PET) wird das Laminat getrocknet und eine geeignete okklusive Trägerschicht zukaschiert. Hierbei wurden, um die nötige Dicke bzw. Wirkstoffbeladung zu erreichen für Beispiel 2-1 drei Schichten zusammenkaschiert (jeweils mit einem geeigneten Gewebe (z.B. PET-Vlies) zur Stabilisierung zwischen den Acrylatschichten) bzw. für 2-2 und 2-3 drei Schichten ohne Gewebe bzw. für 2-4 zwei Schichten bzw. für 2-5 bis 2-8 eine Schicht. Nach Stanzen der TTS werden diese in einem geeigneten Material verpackt, bevorzugt PET.

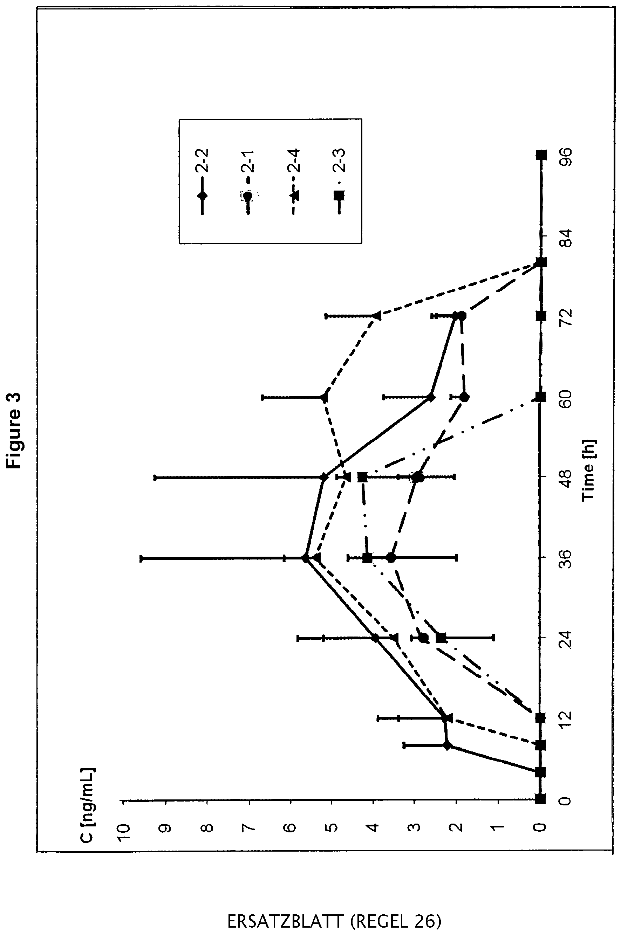

Die erfindungsgemäßen TTS gemäß den Beispielen 2-1, 2-2, 2-3 und 2-4 wurden in vivo (Kaninchen) untersucht. Die Plasmakonzentrationen von Memantin nach dreitägiger Applikation (Entfernung der TTS nach 72 h) sind in Figur 3 abgebildet.

### Beispiel 3:

Die TTS gemäß Beispiel 2-7 wurden in einer klinischen Human-Studie untersucht. Eine Probandengruppe (Gruppe 1) erhielt 3 wirkstoffhaltige Pflaster mit einem Wirkstoffgehalt von jeweils 24,0 mg Memantin berechnet als Base. Nach 24 Stunden wurde eines der Pflaster entfernt. Nach weiteren 24 Stunden wurde ein weiteres Pflaster entfernt. Nach weiteren 24 Stunden wurde das letzte Pflaster entfernt. Eine andere Probandengruppe (Gruppe 2) erhielt 2 wirkstoffhaltige Pflaster mit einem Wirkstoffgehalt von jeweils 24,0 mg Memantin berechnet als Base für 48 Stunden.

Aus den gemessenen Plasmakonzentrationen wurden die pharmakokinetischen Parameter bestimmt und sind in nachfolgender Tabelle zusammengefasst:

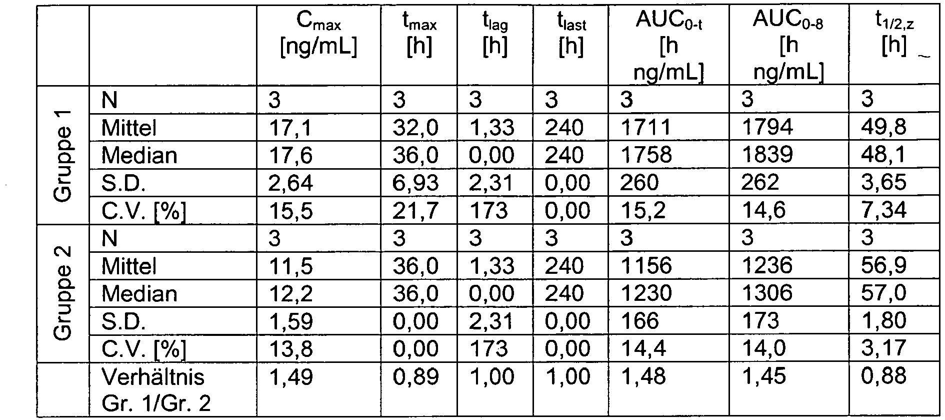

Die Restgehalte an Wirkstoff im TTS nach der Applikation wurden ebenfalls bestimmt und sind für alle 6 Probanden in nachfolgender Tabelle zusammengefasst:

| | | Restgehalt Memantin |
|---|---|---|
| Proband | TTS | 10 cm² TTS [mg/10 cm²] |
| 1 | 1 | 16,569 |
| | 2 | 15,152 |
| | 3 | 14,257 |
| | Mittelwert | 15,326 |
| | Standardabweichung | 1,1656 |
| 2 | 1 | 17,058 |
| | 2 | 15,112 |
| | 3 | 13,500 |
| | Mittelwert | 15,223 |
| | Standardabweichung | 1,7816 |
| 3 | 1 | 17,412 |
| | 2 | 16,865 |
| | 3 | 16,404 |
| | Mittelwert | 16,894 |
| | Standardabweichung | 0,5046 |
| 4 | 1 | 16,193 |
| | 2 | 15,858 |
| | Mittelwert | 16,026 |
| | Standardabweichung | 0,2369 |
| 5 | 1 | 15,330 |
| | 2 | 15,356 |
| | Mittelwert | 15,343 |
| | Standardabweichung | 0,0184 |
| 6 | 1 | 14,810 |
| | 2 | 15,159 |
| | Mittelwert | 14,985 |
| | Standardabweichung | 0,2468 |

## Patentansprüche

1. Memantin in Form der freien Base oder eines physiologisch verträglichen Salzes zur Verwendung in einem therapeutischen Verfahren zur Behandlung oder Vorbeugung der Alzheimer-Erkrankung (Morbus Alzheimer), **dadurch gekennzeichnet, dass** es in einem transdermalen therapeutischen System vorliegt, welches
- eine wirkstoffundurchlässige Trägerschicht;
- mindestens eine wirkstoffhaltige Schicht, welche ein Speichermaterial enthält, in das Memantin in Form der freien Base oder eines physiologisch verträglichen Salzes als Wirkstoff eingebracht ist;
- gegebenenfalls eine Schutzfolie,
- eine tₘₐₓ von mindestens 24 h aufweist, wobei tₘₐₓ als diejenige Zeit definiert ist, nach welcher während der einmaligen oder erstmaligen Applikation des Transdermalen Therapeutischen Systems die maximale Wirkstoffkonzentration im Blutplasma erreicht wird, gemessen ab dem Aufbringen des Transdermalen Therapeutischen Systems auf die Haut, wobei das Speichermaterial
- eine flüssige oder gelförmige Zubereitung umfaßt, in welcher der Wirkstoff teilweise oder vollständig gelöst ist;
und/oder
- eine Matrix auf Basis eines polymeren Materials umfaßt, in welcher der Wirkstoff gelöst oder als Mikrodispersion verteilt vorliegt; und/oder
- ein Lipogel ist, das 10 bis 50 Gew.-% Wirkstoff enthält, und wobei bei aufeinander folgender oder zeitlich versetzter Applikation von zwei oder mehr transdermalen therapeutischen Systemen, die jeweils die gleiche oder eine verschiedene täglich freisetzbare Dosis an Wirkstoff enthalten, für Applikationsintervalle von jeweils unabhängig voneinander mindestens 12 h am behandlungsbedürftigen Individuum in dessen Blutplasma jeweils ab Beginn der erstmaligen Applikation folgende Wirkstoffkonzentrationen erreicht werden:
nach 24 h mindestens 14 ng/ml,
nach 48 h mindestens 27 ng/ml,
nach 60 h mindestens 32 ng/ml,
nach 72 h mindestens 37 ng/ml,
nach 96 h mindestens 44 ng/ml,
nach 144 h mindestens 54 ng/ml,
nach 192 h mindestens 60 ng/ml,
nach 240 h mindestens 63 ng/ml, und
nach 300 h mindestens 65 ng/ml,
wobei die Zeitspanne zwischen dem Entfernen eines Transdermalen Therapeutischen Systems am Ende eines Applikationsintervalls und dem Aufbringen des folgenden Transdermalen Therapeutischen Systems höchstens 10 Minuten beträgt.

2. Memantin zur Verwendung in einem therapeutischen Verfahren gemäß Anspruch 1, wobei für jedes Applikationsintervall die Dauer in h dividiert durch die Anzahl der ggf. gleichzeitig applizierten Transdermalen Therapeutischen Systeme unabhängig voneinander im Bereich von 3 bis 8 liegt.

3. Memantin zur Verwendung in einem therapeutischen Verfahren gemäß Anspruch 1 oder 2, wobei die Wirkstoffkonzentrationen bei Applikation von jeweils einem oder von gleichzeitig mehrerer der Transdermalen Therapeutischen Systeme pro Applikationsintervall erreicht werden.

4. Memantin zur Verwendung in einem therapeutischen Verfahren nach einem der vorstehenden Ansprüche, wobei jeweils ab Beginn der erstmaligen Applikation folgende Wirkstoffkonzentrationen erreicht werden:
nach 24 h höchstens 50 ng/ml,
nach 48 h höchstens 90 ng/ml,
nach 60 h höchstens 110 ng/ml,
nach 72 h höchstens 130 ng/ml,
nach 96 h höchstens 150 ng/ml,
nach 144 h höchstens 180 ng/ml,
nach 192 h höchstens 195 ng/ml,
nach 240 h höchstens 200 ng/ml, und
nach 300 h höchstens 210 ng/ml.

5. Memantin zur Verwendung in einem therapeutischen Verfahren nach einem der vorstehenden Ansprüche, wobei die Wirkstoffkonzentrationen erreicht werden, wenn jedes Applikationsintervall unabhängig voneinander höchstens 72 h beträgt.

6. Memantin zur Verwendung in einem therapeutischen Verfahren nach einem der vorstehenden Ansprüche, wobei
- die über das Applikationsintervall gemittelte Freisetzungsrate des Wirkstoffs im Bereich von 0,2 bis 2,0 mg/h liegt; und/oder
- seine pharmakokinetische Verzögerungszeit t_{lag} höchstens 6 Stunden beträgt.

7. Memantin zur Verwendung in einem therapeutischen Verfahren nach einem der vorstehenden Ansprüche, wobei in dem Transdermalen Therapeutischen System das Speichermaterial zwischen der wirkstoffundurchlässigen Trägerschicht und einem wirkstoffdurchlässigen Membranmaterial angeordnet ist.

8. Memantin zur Verwendung in einem therapeutischen Verfahren nach einem der vorstehenden Ansprüche, wobei der Wirkstoff als physiologisch verträgliches Salz vorliegt.

9. Memantin zur Verwendung in einem therapeutischen Verfahren nach Anspruch 8, wobei die wirkstoffhaltige Schicht zusätzlich mindestens ein basisches Material enthält, dessen Basizität ausreichend ist, um mit dem physiologisch verträglichen Salz des Wirkstoffs unter Freisetzung der freien Base des Wirkstoffs zu reagieren.

10. Memantin zur Verwendung in einem therapeutischen Verfahren nach Anspruch 9, wobei das basische Material ausgewählt ist aus der Gruppe bestehend aus Polymeren mit basischen Seitengruppen, organischen Basen und anorganischen Basen.

11. Memantin zur Verwendung in einem therapeutischen Verfahren nach Anspruch 10, wobei das basische Material ausgesucht ist aus der Gruppe bestehend aus Polymeren auf Acrylatbasis, welche Aminoalkylseitengruppen aufweisen; anorganischen Oxiden oder Hydroxiden; organischen Aminen oder deren Salzen; Alkali- oder Erdalkalialkoholaten oder -carboxylaten.

12. Memantin zur Verwendung in einem therapeutischen Verfahren nach einem der Ansprüche 9 bis 11, wobei die wirkstoffhaltige Schicht 20 bis 90 Gew.-% Speichermaterial, 10 bis 50 Gew.-% Wirkstoff, sowie ggf. 5 bis 40 Gew.-% basisches Material enthält, bezogen auf das Gesamtgewicht der wirkstoffhaltigen Schicht.

## Claims

1. Memantine in the form of the free base or of a physiologically compatible salt for use in a therapeutic method for treating or preventing Alzheimer's disease, **characterized in that** it is present in a transdermal therapeutic system, which has
- a backing layer impermeable to active ingredient;
- at least one active-ingredient-containing layer which comprises a storage material into which memantine in the form of the free base or of a physiologically compatible salt is introduced as active ingredient;
- optionally a release liner,
- a tₘₐₓ of at least 24 h, where tₘₐₓ is defined as the time which has elapsed before the maximum active ingredient concentration in the blood plasma is reached during the one-off or first administration of the Transdermal Therapeutic System, measured starting from the application of the Transdermal Therapeutic System to the skin,
wherein the storage material
- comprises a preparation which is in liquid or gel form and in which the active ingredient is partly or completely dissolved; and/or
- comprises a matrix which is based on a polymeric matrial and in which the active ingredient is dissolved or distributed as a microdispersion; and/or
- is a lipogel which contains from 10 to 50% by weight of active ingredient,
and wherein consecutive or staggered administration of two or more transdermal therapeutic systems, which in each case contain the same or a different daily releasable dose of active ingredient, for administration intervals each lasting at least 12 h independently of one another achieves, in the blood plasma of the individual requiring treatment, the following active ingredient concentrations from the beginning in each case of the first administration:
after 24 h, at least 14 ng/ml,
after 48 h, at least 27 ng/ml,
after 60 h, at least 32 ng/ml,
after 72 h, at least 37 ng/ml,
after 96 h, at least 44 ng/ml,
after 144 h, at least 54 ng/ml,
after 192 h, at least 60 ng/ml,
after 240 h, at least 63 ng/ml, and
after 300 h, at least 65 ng/ml,
wherein the period between the removal of a Transdermal Therapeutic System at the end of an administration interval and the application of the following Transdermal Therapeutic System is no more than 10 minutes.

2. Memantine for use in a therapeutic method according to Claim 1, wherein, for each administration interval, the duration in h divided by the number of the, if applicable, simultaneously administered Transdermal Therapeutic Systems is independently in the range from 3 to 8.

3. Memantine for use in a therapeutic method according to Claim 1 or 2, wherein the active ingredient concentrations are achieved upon administering one Transdermal Therapeutic System per administration interval or upon administering, simultaneously, multiple Transdermal Therapeutic Systems per administration interval.

4. Memantine for use in a therapeutic method according to any of the preceding claims, wherein the following active ingredient concentrations are achieved from the beginning in each case of the first administration:
after 24 h, no more than 50 ng/ml,
after 48 h, no more than 90 ng/ml,
after 60 h, no more than 110 ng/ml,
after 72 h, no more than 130 ng/ml,
after 96 h, no more than 150 ng/ml,
after 144 h, no more than 180 ng/ml,
after 192 h, no more than 195 ng/ml,
after 240 h, no more than 200 ng/ml, and
after 300 h, no more than 210 ng/ml.

5. Memantine for use in a therapeutic method according to any of the preceding claims, wherein the active ingredient concentrations are achieved when each administration interval is, independently of the others, not more than 72 h.

6. Memantine for use in a therapeutic method according to any of the preceding claims, wherein
- the active ingredient release rate averaged over the administration interval is in the range from 0.2 to 2.0 mg/h; and/or
- the pharmacokinetic delay t_{lag} thereof is not more than 6 hours.

7. Memantine for use in a therapeutic method according to any of the preceding claims, wherein in the Transdermal Therapeutic System the storage material is arranged between the active-ingredient-impermeable backing layer and an active-ingredient-permeable membrane material.

8. Memantine for use in a therapeutic method according to any of the preceding claims, wherein the active ingredient is present as a physiologically compatible salt.

9. Memantine for use in a therapeutic method according to Claim 8, wherein the active-ingredient-containing layer additionally comprises at least one basic material, the basicity of which is sufficient to react with the physiologically compatible salt of the active ingredient to release the free base of the active ingredient.

10. Memantine for use in a therapeutic method according to Claim 9, wherein the basic material is selected from the group consisting of polymers having basic side groups, organic bases and inorganic bases.

11. Memantine for use in a therapeutic method according to Claim 10, wherein the basic material is selected from the group consisting of acrylate-based polymers which have aminoalkyl side groups; inorganic oxides or hydroxides; organic amines or salts thereof; alkali metal alcoholates or carboxylates or alkaline earth metal alcoholates or carboxylates.

12. Memantine for use in a therapeutic method according to any of Claims 9 to 11, wherein the active-ingredient-containing layer comprises from 20 to 90 % by weight of storage material, from 10 to 50 % by weight of active ingredient, and optionally from 5 to 40 % by weight of basic material, based on the total weight of the active-ingredient-containing layer.

## Revendications

1. Mémantine sous forme de la base libre ou d'un sel physiologiquement compatible pour une utilisation dans un procédé thérapeutique destiné au traitement ou à la prévention de la maladie d'Alzheimer (démence de type Alzheimer), **caractérisée en ce qu'**elle se trouve dans un système thérapeutique transdermique qui présente
- une couche support imperméable à la substance active ;
- au moins une couche à substance active contenant un matériau de stockage au sein duquel la mémantine est introduite en tant que substance active sous forme de la base libre ou d'un sel physiologiquement compatible ;
- éventuellement un film protecteur,
- un tₘₐₓ d'au moins 24 h, dans laquelle le tₘₐₓ est défini comme étant le temps après lequel, au cours de l'unique ou première application du système thérapeutique transdermique, la concentration maximale de substance active dans le plasma sanguin est atteinte, la mesure s'effectuant à partir du moment où le système thérapeutique transdermique est appliqué sur la peau, dans laquelle le matériau de stockage
- comprend une préparation liquide ou en gel, au sein de laquelle la substance active est partiellement ou complètement dissoute ;
et/ou
- comprend une matrice à base d'un matériau polymère au sein de laquelle la substance active est dissoute ou répartie sous forme de microdispersion ; et/ou
- est un lipogel contenant entre 10 et 50 % en poids de substance active, et dans lequel
les concentrations en substance active ci-dessous sont atteintes au début de la première application, lors de l'application successive ou échelonnée de deux ou plus systèmes thérapeutiques transdermiques contenant respectivement la même dose, ou une dose différente, de substance active pouvant être libérée quotidiennement, pour des intervalles d'application d'au moins 12 h respectivement indépendamment les uns des autres sur un individu nécessitant un traitement, au sein du plasma dudit individu :
au moins 14 ng/ml après 24 h,
au moins 27 ng/ml après 48 h,
au moins 32 ng/ml après 60 h,
au moins 37 ng/ml après 72 h,
au moins 44 ng/ml après 96 h,
au moins 54 ng/ml après 144 h,
au moins 60 ng/ml après 192 h,
au moins 63 ng/ml après 240 h, et
au moins 65 ng/ml après 300 h,
dans laquelle l'intervalle de temps entre le retrait d'un système thérapeutique transdermique à la fin d'un intervalle d'application et l'application du système thérapeutique transdermique suivant est d'au plus 10 minutes.

2. Mémantine pour une utilisation dans un procédé thérapeutique selon la revendication 1, dans laquelle, pour chaque intervalle d'application, la durée en h divisée par le nombre de systèmes thérapeutiques transdermiques éventuellement appliqués simultanément se situe indépendamment les uns des autres dans la plage comprise entre 3 et 8.

3. Mémantine pour une utilisation dans un procédé thérapeutique selon la revendication 1 ou 2, dans laquelle les concentrations en substance active sont atteintes lors de l'application de respectivement un ou de simultanément plusieurs des systèmes thérapeutiques transdermiques par intervalle d'application.

4. Mémantine pour une utilisation dans un procédé thérapeutique selon l'une quelconque des revendications précédentes, dans laquelle les concentrations en substance active ci-dessous sont atteintes respectivement au début de la première application :
au plus 50 ng/ml après 24 h,
au plus 90 ng/ml après 48 h,
au plus 110 ng/ml après 60 h,
au plus 130 ng/ml après 72 h,
au plus 150 ng/ml après 96 h,
au plus 180 ng/ml après 144 h,
au plus 195 ng/ml après 192 h,
au plus 200 ng/ml après 240 h, et
au plus 210 ng/ml après 300 h.

5. Mémantine pour une utilisation dans un procédé thérapeutique selon l'une quelconque des revendications précédentes, dans laquelle les concentrations en substance active sont atteintes lorsque chaque intervalle d'application est indépendamment des autres d'au plus 72 h.

6. Mémantine pour une utilisation dans un procédé thérapeutique selon l'une quelconque des revendications précédentes, dans laquelle
- la vitesse moyenne de libération de la substance active au cours de l'intervalle d'application est comprise entre 0,2 et 2,0 mg/h ; et/ou
- son délai pharmacocinétique t_{lag} est d'au plus 6 heures.

7. Mémantine pour une utilisation dans un procédé thérapeutique selon l'une quelconque des revendications précédentes, dans laquelle le matériau de stockage est disposé dans le système thérapeutique transdermique entre la couche support imperméable à la substance active et un matériau de membrane perméable à la substance active.

8. Mémantine pour une utilisation dans un procédé thérapeutique selon l'une quelconque des revendications précédentes, dans laquelle la substance active est présente sous forme de sel physiologiquement compatible.

9. Mémantine pour une utilisation dans un procédé thérapeutique selon la revendication 8, dans laquelle la couche à substance active contient en outre au moins un matériau basique dont la basicité est suffisante pour réagir avec le sel physiologiquement compatible de la substance active avec libération de la base libre de la substance active.

10. Mémantine pour une utilisation dans un procédé thérapeutique selon la revendication 9, dans laquelle le matériau basique est choisi parmi le groupe constitué de polymères présentant des groupes latéraux basiques, des bases organiques et des bases minérales.

11. Mémantine pour une utilisation dans un procédé thérapeutique selon la revendication 10, dans laquelle le matériau basique est sélectionné parmi le groupe constitué de polymères à base d'acrylate présentant des groupes latéraux aminoalkyle ; d'oxydes ou d'hydroxydes inorganiques ; d'amines organiques ou leurs sels ; d'alcoolates ou de carboxylates alcalins ou alcalino-terreux.

12. Mémantine pour une utilisation dans un procédé thérapeutique selon l'une quelconque des revendications 9 à 11, dans laquelle la couche à substance active contient entre 20 et 90 % en poids de matériau de stockage, entre 10 et 50 % en poids de substance active, et éventuellement entre 5 et 40 % en poids de matériau basique, par rapport au poids total de la couche à substance active.
